# DEMANDE DE BREVET EUROPEEN

(11) **EP 2 051 018 A2**
(43) Date de publication de la demande: **22.04.2009**
(21) Numéro de dépôt: 08305691.1
(22) Date de dépôt: 16.10.2008
(51) Int. Cl.: F24F 3/12, A61L 9/14

(54) **Dispositif d'émission ou de déplacement d'air chaud**

(30) Priorité: 17.10.2007 FR 0707277
(71) Demandeur: Atlantic Industrie, 85000 La Roche sur Yon (FR)
(72) Inventeur: Bony, Yves, 85170, Dompierre/Yon (FR)
(74) Mandataire: Laget, Jean-Loup

(57) **Abrégé**

Un dispositif d'émission ou de déplacement d'air chaud comporte une enveloppe (1) traversée par l'air chaud et présentant une boucle de commande comporte un capteur (4) de détection d'odeur, et au moins un moyen (5 ou 6) d'émission de parfum ou de senteur et/ou de neutralisation ou de correction d'odeur caractérisé par le fait que l'enveloppe contient des moyens (2) de génération d'air chaud agencés sous le moyen (5 ou 6) d'émission de parfum ou de senteur et/ou de neutralisation ou de correction d'odeur, de manière à en assurer la diffusion par effet de cheminée.

Application au contrôle, à la correction ou au maintien de caractéristiques olfactives de l'atmosphère d'un local.

## Description

L'invention est relative à un dispositif d'émission ou de déplacement d'air chaud.

L'invention est particulièrement utile dans son application aux appareils de chauffage, aux convecteurs, radiateurs et autres appareils rayonnants, ainsi qu'aux dispositifs de ventilation mécanique contrôlée, et enfin aux modules échangeurs de chaleur, notamment pour appareils de climatisation ou pompes à chaleur.

Dans tous ces dispositifs connus d'émission ou de déplacement d'air chaud, il existe un risque de dégradation de l'atmosphère par l'émission d'odeur de brûlé lors de la première mise en route ou par calcination de poussières après un arrêt prolongé du dispositif.

En effet, lors de la première utilisation, par exemple par mise sous tension d'éléments chauffants, les graisses et huiles utilisées pour la construction du dispositif sont chauffées ou brûlées, en émettant des odeurs indésirables dans la pièce ou le local à chauffer.

Egalement, lors de périodes d'arrêt importantes, les particules et autres poussières se déposent sur les surfaces génératrices de chaleur. Lors de la remise en service, le réchauffage ou la calcination de ces particules et autres poussières provoque l'émission de mauvaises odeurs correspondantes.

Un premier but de l'invention est de préserver le confort d'un utilisateur, en réduisant l'effet de mauvaises odeurs dues au fonctionnement du dispositif.

Un deuxième but de l'invention est d'améliorer la sensation olfactive de l'atmosphère d'une pièce ou d'un local relié à un dispositif d'émission ou de déplacement d'air chaud.

Un troisième but de l'invention est de mesurer la qualité olfactive de l'atmosphère d'une pièce ou d'un local relié à un dispositif d'émission ou de déplacement d'air chaud.

Le document JP 04 288164 décrit un dispositif d'émission ou de déplacement d'air comportant une enveloppe traversée par l'air d'une pièce ou d'une chambre, avec une boucle de commande comportant un capteur de détection d'odeur, et au moins un moyen d'émission de parfum ou de senteur et/ou de neutralisation ou de correction d'odeur. Ce dispositif comporte une boîte contenant un agent odorant, dont l'ouverture est commandée par un moteur, en cas de détection d'un niveau d'odeur inférieur à un niveau prédéterminé par le capteur de détection d'odeur. En cas de détection d'un niveau d'odeur supérieur à un niveau prédéterminé par le capteur de détection d'odeur, un générateur d'ozone est mis en marche pour désodoriser l'air ambiant de la pièce ou de la chambre, aspiré par un ventilateur.

Le document JP 2002 039627 décrit un appareil de chauffe muni d'un système anti-pollution et comportant un capteur d'odeur présentant deux sensibilités de détection pour mesurer une contamination éventuelle.

Le document JP 2001 087370 décrit un conteneur avec des moyens de neutralisation d'odeurs alcaline et acide, pour désodoriser l'air aspiré dans le conteneur par un ventilateur.

Le document JP 60132623 décrit un purificateur d'air comportant un ventilateur d'aspiration d'air avec filtre anti-poussière, un capteur de détection d'odeur associé à un circuit de conversion et de comparaison à un seuil de mauvaise odeur, pour commander le fonctionnement d'une pompe de pulvérisation de déodorant.

Un quatrième but de l'invention est de proposer un nouveau dispositif d'émission ou de déplacement d'air, de fonctionnement simple et de fabrication économique, adapté dans son application aux appareils de chauffage, aux convecteurs, radiateurs et autres appareils rayonnants, ainsi qu'aux dispositifs de ventilation mécanique contrôlée, et enfin aux modules échangeurs de chaleur, notamment pour appareils de climatisation ou pompes à chaleur.

L'invention a pour objet un dispositif d'émission ou de déplacement d'air chaud, comportant une enveloppe traversée par l'air chaud, présentant une boucle de commande comportant un capteur de détection d'odeur, et au moins un moyen d'émission de parfum ou de senteur et/ou de neutralisation ou de correction d'odeur, caractérisé par le fait que l'enveloppe contient des moyens de génération d'air chaud agencés sous le moyen d'émission de parfum ou de senteur et/ou de neutralisation ou de correction d'odeur, de manière à en assurer la diffusion par effet de cheminée.

Selon d'autres caractéristiques alternatives de l'invention :

Le dispositif comporte un moyen d'émission de parfum ou de senteur et un moyen de neutralisation ou de correction d'odeur.

Le dispositif comporte un circuit de commande portant le capteur de détection d'odeur.

Le circuit de commande est un circuit programmable.

Le circuit de commande comporte un microcontrôleur.

Le microcontrôleur intègre une base de temps.

Le dispositif comporte un ventilateur, une turbine ou moyen analogue de ventilation forcée.

Le capteur de détection d'odeur, ainsi que chaque moyen d'émission de parfum ou de senteur et/ou de neutralisation ou de correction d'odeur, est disposé à proximité de la partie haute de l'enveloppe de manière à interagir avec le courant d'air chaud traversant l'enveloppe

L'invention sera mieux comprise grâce à la description qui va suivre, donnée à titre d'exemple non limitatif en référence aux dessins annexés dans lesquels :
La figure 1 représente schématiquement un dispositif d'émission ou de déplacement d'air selon l'invention.
La figure 2 représente schématiquement un circuit de commande de dispositif selon l'invention.

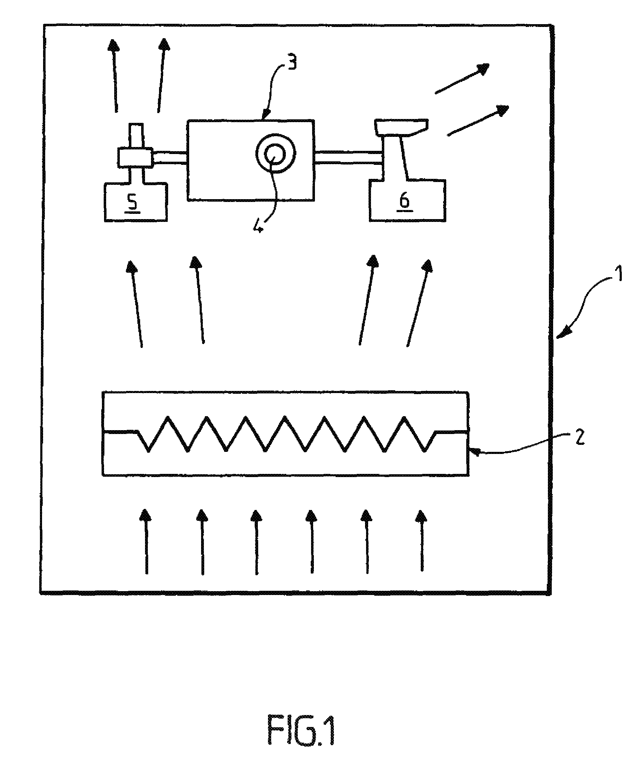

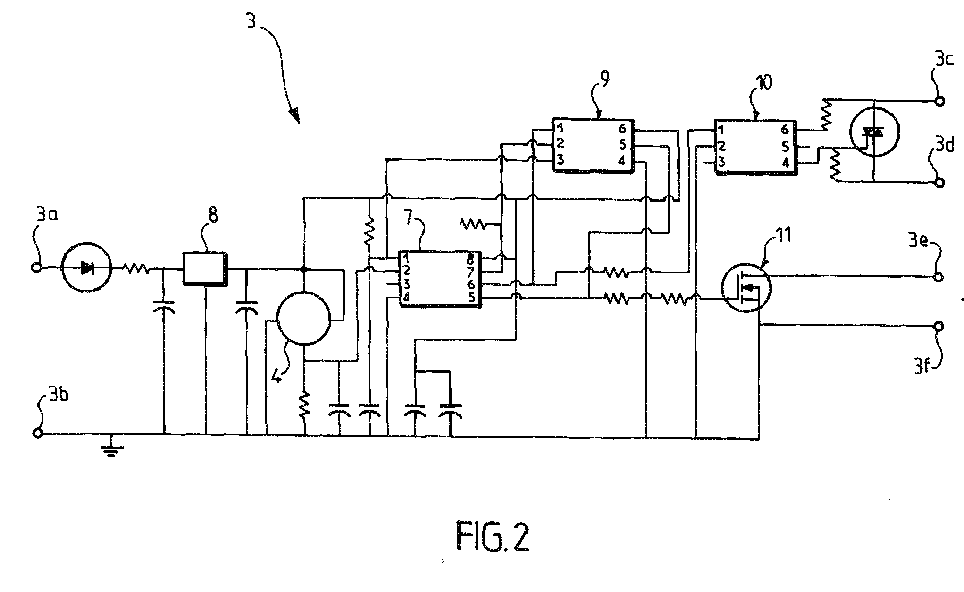

En référence aux figures 1 et 2, les éléments identiques ou fonctionnellement équivalents sont repérés par des chiffres de référence identiques.

Sur la figure 1, un dispositif selon l'invention comporte une enveloppe 1 contenant des moyens 2 de génération ou de déplacement d'air chaud, par exemple un corps de chauffe 2.

L'enveloppe 1 comporte en partie haute un circuit 3 de commande portant ou relié à un capteur 4 de détection d'odeur.

Le circuit 3 de commande est de préférence programmable et apte à commander un moyen 5 d'émission de parfum ou de senteur et/ou à déclencher un moyen 6 de neutralisation ou de correction d'odeur.

Les éléments 3 à 6 sont disposés au-dessus des moyens 2 de génération ou de déplacement d'air chaud, de manière à diffuser les produits émis par les moyens 5 et/ou 6 et de manière à détecter les odeurs plus efficacement au moyen du capteur 4 de détection d'odeur.

Ainsi, grâce à l'invention, la qualité olfactive de l'atmosphère d'une pièce ou d'un local peut être corrigée ou améliorée de manière ponctuelle ou de manière programmée.

Ainsi, la détection d'odeur par le capteur 4 est analysée quantitativement par le circuit 3 de commande et comparée à un seuil prédéterminé, avant de déclencher éventuellement le moyen 6 de neutralisation ou de correction d'odeur.

Egalement, en cas de mauvaise odeur subite correspondant à un pic de détection, le circuit 3 de commande peut actionner le moyen 5 d'émission de parfum ou de senteur.

Une diffusion à intervalles réguliers ou une diffusion cyclique de senteur ou de parfum peut être effectuée par le moyen 5 d'émission de parfum ou de senteur, sur commande du circuit programmable 3 pour assurer un état permanent d'une qualité d'atmosphère recherchée.

Enfin dans le cas où le circuit programmable 3 comporte une horloge ou une base de temps, notamment dans le cas où le circuit programmable 3 comporte un microcontrôleur, une diffusion d'agent neutralisant ou de parfum ou de senteur peut être programmée après chaque durée d'arrêt du dispositif supérieure à une durée prédéterminée, par exemple de l'ordre de deux mois.

Bien entendu, l'invention couvre également toute autre correction ou stabilisation olfactive d'atmosphère au moyen d'un programme préétabli chargé dans un microcontrôleur d'un circuit 3 de commande, en assurant ainsi une automatisation des actions correctrices ou stabilisatrices.

L'utilisation du capteur 4 de détection d'odeur permet d'effectuer un asservissement précis des actions correctrices ou stabilisatrices, en évitant les surdosages et les consommations inefficaces d'agent neutralisant ou de parfum ou de senteur.

La disposition des moyens 5 et 6 d'émission de parfum ou de senteur ou d'agent neutralisant dans le courant d'air chaud permet un brassage et un mélange rapides avec l'atmosphère de la pièce ou du local, ce qui améliore également la réactivité du capteur 4 de détection d'odeur et diminue le temps de réponse et le retour d'information de la boucle d'asservissement correspondante.

Lorsque le dispositif selon l'invention comporte un ventilateur, une turbine ou un autre moyen de ventilation forcée, le brassage et le mélange sont encore accélérés par la ventilation forcée et par effet de cheminée.

Sur la figure 2, un circuit 3 de commande de dispositif selon l'invention comporte un capteur 4 de détection d'odeur, un microcontrôleur 7 intégrant une base de temps, un régulateur de tension 8, un convertisseur série parallèle 9, un module 10 optocoupleur et un commutateur 11 d'alimentation.

Le circuit 3 de commande comporte deux bornes 3a, 3b d'alimentation et quatre bornes 3c, 3d, 3e et 3f de sortie pour commander les moyens 5 et 6 d'émission de parfum ou de senteur et de neutralisation ou de correction d'odeur.

Ainsi, en chargeant un programme approprié dans le microcontrôleur 7, l'invention permet de mesurer ou détecter les caractéristiques olfactives d'une pièce et d'effectuer les actions correctrices ou stabilisatrices d'atmosphère prévues par le programme.

L'invention décrite en référence à un mode de réalisation particulier n'y est nullement limitée, mais couvre au contraire toute modification de forme et toute variante de réalisation dans le cadre et l'esprit de l'invention.

## Revendications

1. Dispositif d'émission ou de déplacement d'air chaud, comportant une enveloppe (1) traversée par l'air chaud, présentant une boucle de commande comportant un capteur (4) de détection d'odeur, et au moins un moyen (5 ou 6) d'émission de parfum ou de senteur et/ou de neutralisation ou de correction d'odeur, **caractérisé par le fait que** l'enveloppe contient des moyens (2) de génération d'air chaud agencés sous le moyen (5 ou 6) d'émission de parfum ou de senteur et/ou de neutralisation ou de correction d'odeur, de manière à en assurer la diffusion par effet de cheminée.

2. Dispositif selon la revendication 1, **caractérisé par le fait que** le dispositif comporte un moyen (5) d'émission de parfum ou de senteur et un moyen (6) de neutralisation ou de correction d'odeur.

3. Dispositif selon la revendication 1 ou la revendication 2, **caractérisé par le fait que** le dispositif comporte un circuit (3) de commande portant le capteur (4) de détection d'odeur.

4. Dispositif selon la revendication 3, **caractérisé par le fait que** le circuit (3) de commande est un circuit programmable.

5. Dispositif selon la revendication 3 ou la revendication 4, **caractérisé par le fait que** le circuit (3) de commande comporte un microcontrôleur (7).

6. Dispositif selon la revendication 5, **caractérisé par le fait que** le microcontrôleur (7) intègre une base de temps.

7. Dispositif selon l'un quelconque des revendications précédentes, **caractérisé par le fait que** le dispositif comporte un ventilateur, une turbine ou moyen analogue de ventilation forcée.

8. Dispositif selon l'une quelconque des revendications précédentes, **caractérisé par le fait que** le capteur (4) de détection d'odeur, ainsi que chaque moyen (5 ou 6) d'émission de parfum ou de senteur et/ou de neutralisation ou de correction d'odeur, est disposé à proximité de la partie haute de l'enveloppe de manière à interagir avec le courant d'air chaud traversant l'enveloppe (1).
